# EUROPÄISCHE PATENTSCHRIFT

(11) **EP 2 087 863 B1**
(45) Veröffentlichungstag und Bekanntmachung des Hinweises auf die Patenterteilung: **26.10.2011**
(21) Anmeldenummer: 09001569.4
(22) Anmeldetag: 05.02.2009
(51) Int. Cl.: A61F 5/058, A61F 5/10

(54) **Finger-Orthese**
Finger orthosis
Orthèse de doigt

(30) Priorität: 08.02.2008 DE 102008008257; 09.06.2008 DE 102008027439
(43) Veröffentlichungstag der Anmeldung: 12.08.2009
(73) Patentinhaber: Weskott, Christina, 50858 Köln (DE)
(72) Erfinder: Weskott, Christina, 50858 Köln (DE)
(74) Vertreter: Schäfer, Horst

(56) Entgegenhaltungen:
- EP-A- 1 813 233
- DE-A1- 19 651 912
- FR-A- 752 041
- GB-A- 1 326 351
- US-A- 4 932 396
- US-A- 5 882 323
- US-A1- 2004 144 389

## Beschreibung

Die Erfindung betrifft Orthesen für mindestens eine Extremität, insbesondere für Finger, aber auch für Zehen.

Orthesen für eine Extremität, insbesondere für Finger einer Hand (unabhängig ob für einen Daumen und einen anderen Finger, der rechten oder der linken Hand) sind orthopädische Hilfs- und Heilmittel, die im wesentlichen Aufgaben haben, wie: einem Gelenk Halt zu geben, ein Überstrecken eines Gelenks zu korrigieren, schmerzhafte Fehlstellungen zu verhindern, Strecken der Glieder zu Korrektur oder Abduktionen, insbesondere ulnare Deviationen zu korrigieren oder zu verkleinern. Als Stand der Technik soll beispielhaft hier erwähnt werden: US 4,270,528 oder EP 1813233 A1.

Aus der WO 2004-002377 A1 ist eine flexible Orthese mit einer längsverlaufenden Unterbrechung bekannt geworden. Die Unterbrechung ist mit einem Verschluss überbrückbar und verschließbar. Die Anpassbarkeit der Orthese an verschiedene Finger-Ring-Größen wird durch die Elastizität des Materials und durch die Ausbildung des Verschlusses ermöglicht, wonach beispielsweise zwölf verschiedene Größen einstellbar sind. Nach Anlegen der Orthese an einen Finger werden zwei Ringbügel elastisch zusammengebogen und der Verschluss geschlossen. Die Anpassung an die Finger-Ring-Größe erfolgt durch die vom Verschluss vermittelte elastische Spannung. Der Einsatz dieser Orthese aus elastischem Material ist mit mechanischem Druck auf das Gelenk verbunden, was nicht immer therapeutisch angezeigt ist. Weiterhin hat die Verwendung von elastischem Material, welches beim An- und Ablegen der Orthese gedehnt wird, den Nachteil der Verschleißanfälligkeit.

Aus Gründen, die in der unterschiedlichen Gestalt der Finger, Glieder oder Hände von Patienten und/oder der orthopädischen Fehlstellungen oder einer Erkrankung liegen, sind Orthesen, die aus einem relativ starren Werkstoff, insbesondere aus Schmucklegierungen, hergestellt werden, von dem Nachteil behaftet, dass praktisch für jeden Patienten eine individuelle Einzelanfertigung vorgenommen werden muss.

Die Aufgabe der vorliegenden Erfindung besteht darin, eine verbesserte Orthese zur Verfügung zu stellen, welche für die Serienfertigung und für einfaches Anpassen vorbereitet ist.

Die Lösung der Aufgabe wird in Nebenansprüchen formuliert. Weitergehende vorteilhafte Ausgestaltungen sind in den jeweiligen Unteransprüchen aufgeführt.

In der DE 19651912 A ist eine Orthese aus thermisch verformbarem Material gezeigt. Das Material ist anschmiegsam und weich. Zur Befestigung oder zur Herstellung eines korrekten Sitzes an einer Extremität muss die Orthese zusätzlich mit versteifenden Elementen oder Verschlüssen versehen werden.

Es ist eine Orthese mit zwei Ringbügeln bekannt (FR 752 041), zwischen denen ein gerader Steg ausgebildet ist. Das Ausgangsmaterial für diese Orthese ist Flachmaterial, welches endständig je zwei gleich lange Laschen hat. Die Orthese wird an einen Finger dadurch angepasst, dass jeweils die beiden Laschen zu einer Ringform verbogen werden. Dieses Dokument FR 752 041 stellt der nächste Stand der Tecknik dar.

Der Nachteil dieser Orthese besteht darin, dass eine weitere Umformung des Flachmaterials dadurch begrenzt ist, dass das Material bei starker Verformung ermüdet und bricht.

Die Aufgabe der Erfindung besteht darin, eine verbesserte Orthese zur Verfügung zu stellen , welche für die Serienfertigung und für einfaches Anpassen vorbereitet ist.

Die Lösung der Aufgabe wird im Hauptanspruch formuliert. Weitergehende vorteilhafte Ausgestaltungen sind in den Unteransprüchen aufgeführt.

Der Kern der Erfindung liegt darin, dass die Orthese im Bereich mindestens eines Ringbügels und/oder im Bereich des Stegs eine durchgehende, vorzugsweise als Spalt ausgebildete Unterbrechung aufweist und die Orthese oder zumindest einzelne Elemente der Orthese aus plastisch verformbarem Material ausreichender Duktilität hergestellt ist, um während der Anpassung an ein Gelenk plastischen Verformungen ohne Verlust der Festigkeit Stand zu halten.

Als Materialien können Kunststoff, Metall (Stahl, Edelmetall) oder Verbundwerkstoff vorgesehen sein. Eine plastische Verformung kann auch unter Anwendung erhöhter Temperatur vorgenommen werden. Als Werkstoffe können Materialien mit guten funktionellen Eigenschaften, wie dauerhafte optische, chemische oder physikalische (Licht, Temperatur) Beständigkeit oder Haltbarkeit, sowie gute Reinigungsfähigkeit und Handhabbarkeit eingesetzt werden. Aber es ist auch möglich, Werkstoffe nach ästhetischen Gesichtspunkten auszuwählen. Kombinationen von Werkstoffen können dabei vorteilhafte Synergien der Wirkungen erzeugen, welche ebenfalls von der Erfindung umfasst sind. Bevorzugt werden Edelmetalllegierungen und Schmuckmetall-Legierungen verwendet.

Im Folgenden soll die Anwendung einer Orthese ausführlich für Finger oder Daumen beschrieben werden, wodurch jedoch keine Einschränkung auf Finger oder Daumen vorgenommen wird, weil Begriffe wie Zehen und/oder Fuß nicht verwendet werden.

Mit der Orthese wird eine Stützfunktion eines Gelenks, insbesondere Fingergelenk gewährleistet, ohne dass die Orthese vom Gelenk und somit auch vom Finger abrutschen kann, wobei auf Befestigungsbänder verzichtet werden kann. Die Orthese wird für Gelenke mit krankhaften Versteifungen angepasst. Trotz Starrheit der Ausbildung der Orthesen und Fixierung eines oder mehrerer Gelenke können Finger, insbesondere die gesamte Hand greiffähig bleiben.

Die Orthese ist etwa zylindrisch ausgebildet mit einer Längserstreckung, die insbesondere größer ist als die Längserstreckung eines Gelenks eines Fingers. In ihrer inneren Form ist die Orthese einer Außenform eines Fingers und des Gelenks nachgebildet, wobei insbesondere die Winkellagen der Ringbügel gegeneinander geneigt sein können, um damit eine Winkelstellung der beiden am Gelenk befindlichen Fingerglieder, die von der gestreckten Lage (0° Winkel) abweicht, einzustellen. Die Ringbügel befinden sich endständig und kommen distal und proximal eines Gelenks zu liegen. Der Steg liegt unterhalb (fingerinnenseitig) des Gelenks. Der Steg kann in seiner Lage mittig zwischen den Ringbügeln ausgebildet sein. Die Ringbügel erstrecken sich oberhalb der von dem Steg gebildeten Ebene. Der Breite des Stegs kann zwischen einer schmalen und einer breiten Form variieren. Bei einem schmal ausgeführten Steg haben die Ringbügel im Bereich des Stegs (fingerinnenseitig) ihre größte Öffnung. Hierdurch wird dem Finger eine bestimmte, größere Beweglichkeit eingeräumt. In einer alternativen Ausbildung kann der Steg verbreitert gestaltet sein, so dass er im Bereich des Fingergelenks eine besondere Stützfläche bildet und die Beweglichkeit des Fingers im Gelenk eingeschränkt ist. Erfindungsgemäß ist der Steg doppelt ausgeführt, wodurch die beiden Stege seitlich des Gelenks (also nicht fingerinnenseitig) zu liegen kommen.

Die Orthese ist vornehmlich derart ausgebildet, dass beide Ringbügel bei Festlegung der Orthese an einem Finger auf einer Außenseite eines Fingergelenks eine Einfassung für das Fingergelenk bilden und dort das Fingergelenk umschließen.

Einer der Ringbügel, insbesondere der proximale Ringbügel kann in seiner parallel zur Längserstreckung liegenden Breite länger ausgebildet sein als die entsprechende Breite des anderen, also distalen Ringbügels.

Der Steg kann zumindest teilweise zwischen den beiden hintereinanderliegenden Ringbügeln als verbreiterte integrierte Stützschiene ausgebildet sein.

Bevorzugte Ausgestaltungen sollen im folgenden dargestellt werden, wobei die Merkmale der Ausführungsformen einzeln oder in Kombination miteinander verwirklicht sein können.

Die als schmaler Spalt ausgebildete Unterbrechung teilt einen Ringbügel in einen linken und einen rechten Flügel, oder durch die Unterbrechung im Steg wird die Orthese in zwei Hälften unterteilt. Die Breite des Spalts ist in Verhältnis zum Gesamtumfang des Bügels relativ klein, aber doch noch genügend breit, so dass ein Bügel (die Einführöffnung) in seinem in Finger-Ring-Größe gemessenen Durchmesser um eine halbe bis ganze Finger-Ring-Größe vergrößerbar oder verkleinerbar ist.

Durch plastische Verformung ist die Orthese an den Finger und an mindestens ein Fingergelenk anpassbar. Die Verformung kann derart sein, dass die Breite der Unterbrechung verringert oder vergrößert wird, oder bei gleichbleibender Breite eine Verringerung oder Vergrößerung des Durchmessers vorgenommen wird. Hierzu sind besonders Materialien geeignet, die eine genügende Duktilität aufweisen, und einige plastische Verformung ohne Verlust der Festigkeit erlauben.

Durch plastische Verformung wird die Orthese in eine unter normalen Nutzungsbedingungen unveränderbare Form gebracht. Während des Sitzes der Orthese an einem Finger soll die Orthese keine merkliche elastische Verformung erfahren, und keine weitere plastische Verformung erleiden können.

Eine Orthese kann im besonderen für die therapeutische Anwendung an einem Daumen ausgebildet sein. Eine solche Daumen-Orthese hat einen distalen und einen proximalen Ringbügel und einen Steg, wobei der Steg mit jedem der beiden Ringbügel eine ringförmige Öffnung zur Einführung des Daumens bildet und die Daumen-Orthese durch das Zusammenwirken beider Ringbügel auf der der Handaußenfläche zugeordneten Außenfläche des Daumens mit dem Steg auf der der Handinnenfläche zugeordneten Innenfläche des Daumens in einer Endlage am Daumengrundgelenk festlegbar ist, wobei beide Ringbügel eine Gelenkeinfassung bilden, welche das Daumengrundgelenk auf der Handaußenfläche (Daumenaußenfläche) umschließt. Vorzugsweise ist der proximale Ringbügel an einer für den Daumen vorgesehenen Orthese verlängert ausgebildet, so dass der Ringbügel eine zum Daumensattelgelenk auslaufende Stützfläche bildet.

Es können eine oder zwei Unterbrechungen ausgebildet sein, die je nach orthopädischer Situation oder aus praktischen Erwägungen unterschiedliche Lagen an der Orthese haben können. Beispielsweise können sie längs der Einsteckrichtung des Fingers verlaufen, sie können zur Längsachse der Orthese symmetrisch oder asymmetrisch liegen oder sie können schraubenförmig um die Längsachse der Orthesen verlaufen.

Die Ringbügel können derart am Steg ausgebildet sein, dass sie zwei für einen Finger hintereinanderliegende Fingereinführöffnungen bilden. Die beiden Ringbügel werden dann als vorderer und hinterer Ringbügel bezeichnet. Der distale Ringbügel liegt der Fingerspitze näher, der proximale liegt der Fingerspitze fern.

Der Steg kann zumindest teilweise zwischen den beiden hintereinanderliegenden Ringbügeln als verbreiterte integrierte Stützschiene ausgebildet sein.

Der als verbreiterte Stützschiene ausgebildete Steg kann eine Aufweitung aufweisen, die als Gelenkeinfassung auf dem Finger zu liegen kommt.

Die Ringbügel können auch derart am Steg ausgebildet sein, dass sie für zwei oder mehrere Finger je eine Fingereinführöffnung bilden. Die Ringbügel liegen dann - etwa in einer Ebene - nebeneinander, statt hintereinander. Eine solche Orthese dient vornehmlich der Begrenzung einer Ulnardeviation eines oder mehrerer Finger.

Die Orthese ist vorzugsweise einstückig ausgebildet. Allerdings kann sie auch mehrteilig ausgebildet sein und Gelenkmittel aufweisen, z.B. ein Scharnier an einem Ringbügel und/oder im Bereich des Stegs. Die Gelenkmittel können an einem Ringbügel oder im Bereich des Stegs vorhanden sein. Mit einem Scharnier an einem Flügel (an einem Gelenk aufklappbar geteilt) lässt sich ein Ringbügel öffnen und schließen. Bei einem Gelenkmittel im Bereich des Stegs ist der Ringbügel relativ zum Steg verstellbar.

In Verbindung mit einem Gelenkmittel können Verschluss- oder Rastmittel zur Arretierung im geschlossenen Zustand vorhanden sein, die zur Arretierung eines angelenkten Ortheseteils im geschlossenen Zustand dienen. Als Verschlussmittel können im Goldschmiedehandwerk bzw. in der Schmuckindustrie bekannte Verschlüsse, wie Steckverschluss, Ösenverschluss, Schnappverschluss, Klettbandverschluss, oder Magnetverschluss eingesetzt sein. Dabei können Verriegelungshaken, federnde Ösen usw. verwendet werden. Die Verschlussmittel haben zwei einander zugewandte Kupplungsteile. Bei einem Magnetverschluss sind in jedem Kupplungsteil Hochleistungsmagnete, beispielsweise Selten-Erd-Kobalt-Magnete vorhanden.

Zumindest flächenhafte Bereiche einzelner Elemente der Orthese können ergonomische Ausformungen aufweisen, bzw. es können filigrane Ausnehmungen ausgebildet sein.

Wenigstens ein Flügel eines Ringbügels kann klappbar angelenkt sein und wenigstens ein Verschluss- oder Rastmittel für die Arretierung aufweisen. Das Verschlussmittel kann eines der zuvor schon erwähnten Verschlüsse sein. Das Verschlussmittel kann Rastmittel umfassen, mit welchem das angelenkte, bewegliche Teil in einer festen Endlage arretierbar ist.

Mit den vorgestellten Orthesen wird erreicht, dass der oder die Finger während des Tragens in einer physiologischen Stellung verbleiben kann/können. Es wird insbesondere vermieden, dass ein Finger unnatürlich und zwangsweise abgespreizt gehalten wird, was mit erheblichen Einschränkungen der Bewegungsfähigkeit verbunden wäre. Somit bleiben die Bewegungsmöglichkeiten des Fingers erhalten, so dass die Muskulatur des Fingers weiter gefordert wird und ein kontraproduktiver Muskelabbau vermieden werden kann. Beim Tragen der Orthese ist eine dauerhafte Stabilisierung des Fingers und eine Entlastung kranker Gelenke gewährleistet. Dabei kann erfindungsgemäß auf störende Bandbefestigungen verzichtet werden.

Fig. 1 zeigt eine erfindungsgemäße Orthese in Trageposition an einem Zeigefinger, mit Abwicklung. Fig. 2 - Fig. 7 zeigen tecknischen Merkmalen, die zum Verständnis der Erfindung dazu tragen.

Es zeigen im Einzelnen:
Fig. 1: eine Orthese :
Fig. 2: eine erste Daumen-Orthese in Trageposition an einem Daumen, mit Abwicklung;
Fig. 3: eine zweite Daumen-Orthese in Trageposition an einem Daumen, mit Abwicklung;
Fig. 4: eine perspektivische Draufsicht auf eine Orthese;
Fig. 5: eine Ansicht in Richtung der Längsachse einer Orthese,
Fig. 6: eine Schemazeichnung mit einer Vielzahl möglicher Lagen von Unterbrechungen und
Fig. 7: Ansicht einer mehrteiligen Orthese.

Die Figuren beziehen sich auf Orthesen für Finger oder Daumen, wobei jedoch keine Einschränkung nur auf Finger oder Daumen vorgenommen wird.

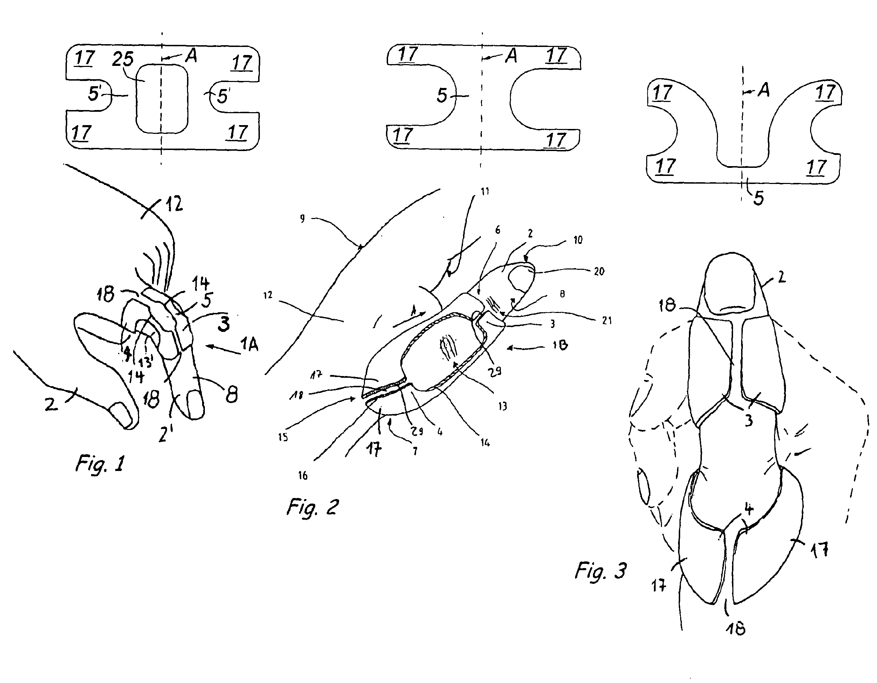

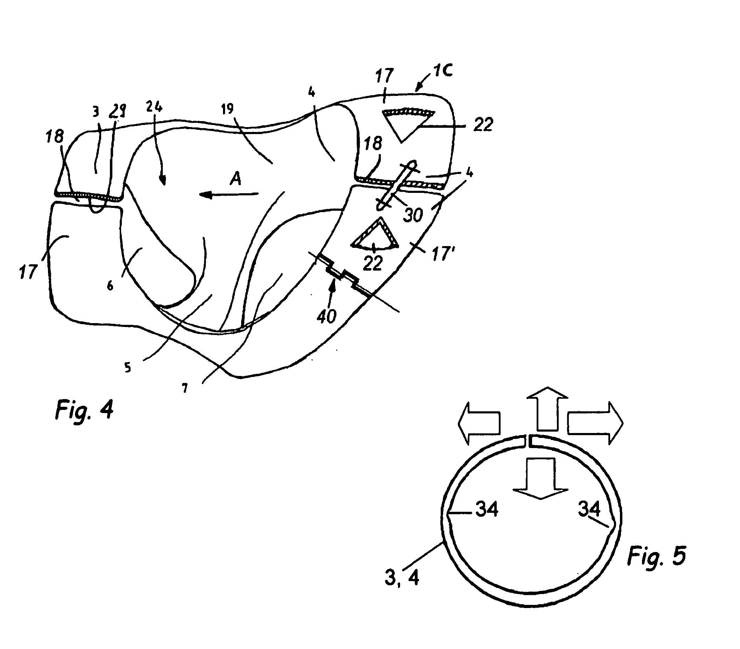

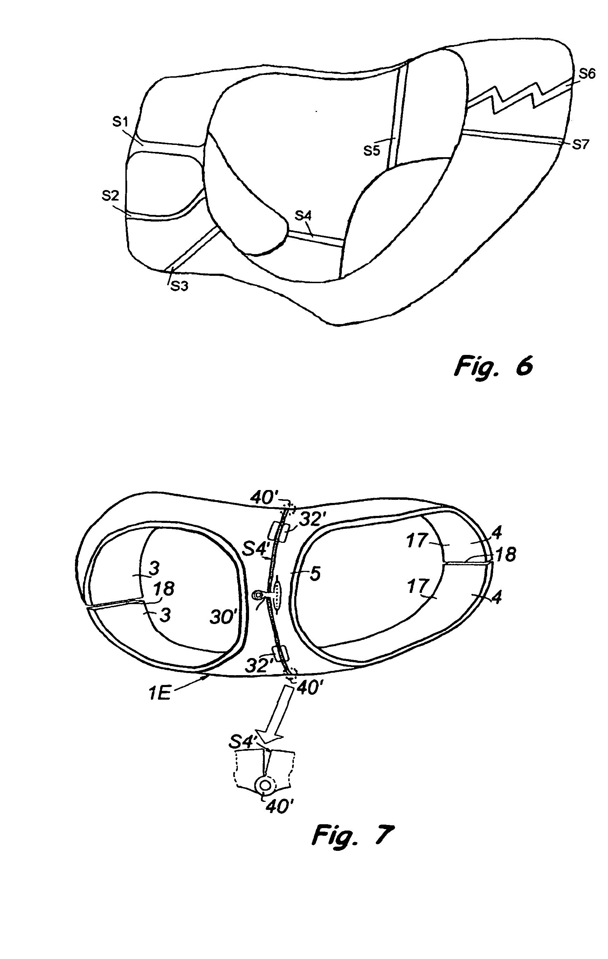

Mit Ausnahme der Orthesen in den Figuren 4 und 7 sind die gezeigten Orthesen 1A, 1B, 1C einstückig ausgebildet sind. Sie haben eine zylindrische oder kegelstumpfartige Form mit einer distalen (vorderen oder oberen) 6, 6' und einer proximalen (hinteren oder unteren) 7,7' ringförmigen Öffnung zum Hindurchstecken eines Fingers. Die genannten Öffnungen werden von je einem Ringbügel 3,4 (3',4') umrandet. Beide Ringbügel werden von einem Steg 5 (in Fig. 1 ein Doppelsteg 5') verbunden. Die Ausbildung des Stegs 5 ist je nach orthopädischem Erfordernis unterschiedlich breit gestaltet. In den Figuren 2 und 3 befindet sich der Steg 5 (unsichtbar) auf der Innenseite des Daumens 2 und ist relativ schmal ausgebildet. Erfindungsgemäß ist der Steg 5 breit ausgebildet und weist innerhalb des Stegs eine Öffnung auf. Eine solche Öffnung dient dann als Gelenkeinfassung 14. Die **Fig. 6** zeigt zusammenfassend schematisch eine Vielzahl möglicher Lagen von Unterbrechungen (S 1 bis S7).

Erfindungsgemäß weist mindestens ein Ringbügel 3,4 eine Lücke, Spalt oder Unterbrechung 18 auf. Mit den Ausbildungen einer Unterbrechung in mindestens einem Ringbügel 3,4 entstehen am Ringbügel jeweils linke und rechte Flügel 17. Durch die in den Orthesen gebildeten Unterbrechungen 18 sind die Orthesen in vorteilhafter Weise durch plastische Verformung des Materials zur Vergrößerung oder Verkleinerung der Fingereinführöffnungen 6,7 an einen Finger 2, 2' anpassbar.

Die Lage der Unterbrechungen kann unterschiedlich sein. In den meisten Figuren sind die Unterbrechungen 18 in jedem Ringbügel vorhanden. In den Formen der Figuren 1 bis 3 verlaufen sie etwa in einer Verbindungslinie zwischen beiden Ringbügeln in Längsrichtung (A), somit parallel zum eingeführten Finger 2' (oder Daumen 2). Jede andere Art der Ausbildung der Unterbrechung 18 soll jedoch auch von der Erfindung erfasst sein.

In den Figuren 1 bis 3 sind oberhalb der Figurendarstellungen jeweils Material-Abwicklung (flach in eine Ebene gelegt) der Orthese dargestellt. Die Abwicklungen zu Fig. 1 zeigt, dass zwei Stege 5' vorhanden sind, so dass auf der Innenseite des Fingers 8 eine Öffnung 25 in Längsrichtung A der Orthese entsteht. Mit Längsrichtung ist dabei die Einsteckrichtung A (siehe Fig. 4) des Fingers gemeint.

In den Fig. 2 und 3 ist der Steg 5 in unterschiedlicher Lage zu den Ringbügeln (Flügeln 17) ausgebildet. Die Abwicklung zu Fig. 2 zeigt eine Lage des Stegs 5, bei der der Steg mittig zwischen den Ringbügeln liegt. Die Abwicklung zu Fig. 3 zeigt eine Lage des Stegs 5, wo der Steg endständig angeordnet ist.

Die Ringbügel bilden - insbesondere nach Fig. 2 - eine Gelenkeinfassung 14, welche auf dem Finger zu liegen kommt. Die Orthese liegt in der Regel in der Trageposition an einem Fingergelenk 13' fest an. Die Gelenkeinfassung 14 umfasst das Fingergelenk 13' auf der Außenfläche 8 des Fingers 2'. Die Bewegung des Fingergelenks 13' ist bei einer nach innen zur Innenfläche der Hand 12 gerichteten Beugung begrenzt. Nach außen hin wird das Strecken der das Gelenk 13' bildenden Fingerglieder durch die starre Anordnung der Ringbügel 3, 4, zwischen welchen der Finger 2' einliegt, im Verhältnis zum Steg 5 verhindert.

Indem zumindest ein Ringbügel 3, 4 oder der Steg eine durchgehende Unterbrechung 18 aufweist, wird in vorteilhafter Weise erreicht, dass die Orthesen in Serienfertigung angefertigt werden können. Die Ringbügel 3, 4 sind dabei so geteilt, dass zwei Flügel 17 entstehen, wobei es nicht auf die Form oder Ausbildung der Unterbrechung 18 ankommt, sondern lediglich darauf, dass beide Flügel 17 relativ zueinander auseinander gebogen oder zusammengepresst werden können.

Erfindungsgemäß werden mit der Ausführungsform unterschiedliche Größen (Finger-Ring-Größen) als Rohling herstellbar. Individuelle Anpassungen können am Patienten unmittelbar durch Veränderungen an den Flügeln 17 (oder im Steg) vorgenommen werden. Die Anpassung an die ergonomischen Erfordernisse sind somit einfach realisierbar, wobei übliche Kleinwerkzeuge für die Materialbearbeitung eingesetzt werden können.

Die Fig. 2 zeigt eine Orthese 1B speziell für einen Daumen, in der vorgesehenen Tragposition. Die Orthese 1B weist einen distalen Ringbügel 3, einen proximalen Ringbügel 4 sowie einen, auf der Innenseite des Daumens liegenden Steg 5 auf. Wie auch in Fig. 4 sichtbar bildet der Steg 5 mit jedem der beiden Ringbügel 3,4 eine ringförmige Öffnung 6,7, durch welche der Finger (Daumen) beim Anlegen der Orthese nacheinander durchgesteckt werden kann. Die Ringbügel 3, 4 liegen in dem in Fig. 2 dargestellten, angelegten Zustand der Orthese auf der Außenfläche 8 des Daumens 2, welche der Handaußenfläche 9 zugeordnet ist, während der Steg 5 auf der entgegengesetzten Innenfläche 10 des Daumens 2 anliegt, welche der Handinnenfläche 11 zugeordnet ist. Die Daumen-Orthese 1B sitzt durch das Zusammenwirken der beiden Ringbügel 3, 4 auf der Daumen-Außenfläche 8 mit dem Steg 5 auf der Innenfläche 10 des Daumens 2 in der Trageposition. Die Trageposition wird dabei durch die Endlage des Stegs 5 in der Beuge des Daumens 2 zwischen Hand 12 und Daumen 2 am Daumengrundgelenk 13 festgelegt. Bei der Daumen-Orthese 1B bleibt die Wirkung einer Gelenkeinfassung 14 des Daumengrundgelenks 13 auf der Außenfläche 8 des Daumens 2 erhalten, wobei das Daumengrundgelenk 13 durch den Steg 5, welcher auf der Innenfläche 10 des Daumens 2 anliegt, in einer leicht gebeugten Haltung gehalten wird.

In Fig. 2 ist der proximale Ringbügel 4 in seiner parallel zur Längserstreckung liegenden Breite länger ausgebildet als die entsprechende Breite des distalen Ringbügels 3. Damit bildet der proximale Ringbügel 4 an einer für einen Daumen vorgesehenen Orthese 1B eine zum Daumensattelgelenk 15 auslaufende Stützfläche 16.

Speziell beim Daumen **(****Fig. 2****)** ist die Situation so, dass beim Greifen der Hand 12 der am Daumensattelgelenk 15 wirksame Kraftwinkel stets ausreichend klein bleibt und somit die das Daumensattelgelenk 15 bildenden Fingerglieder ebenfalls in einer leichten Beuge bleiben. Damit wird das Daumensattelgelenk 15 stabilisiert. Die Stützfunktion der Orthese 1B am Daumensattelgelenk 15 wird durch die erwähnte, am proximale Ringbügel 4 ausgebildete Stützfläche 16 unterstützt. Die Stützfläche 16 erstreckt sich als angeformte Verbreiterung der Flügel des proximalen Ringbügels 4 auf der Außenfläche 8 des Daumens 2 in Richtung des Daumensattelgelenks 15 und ist mit einer konkaven Wölbung an die Ergonomie der Hand 12 angepasst. Die Bewegungsfreiheit des Daumens 2 wird dabei weiterhin durch die Gelenkeinfassung 14 gewährleistet, welche ein Ausweichen des Daumengrundgelenks 13 beim Beugen ermöglicht.

In Fig. 3 wird eine Orthese dargestellt, die ebenfalls für die Daumen - wie in Fig. 2 - angefertigt ist, jedoch die Besonderheit aufweist, dass der distale Ringbügel 3 besonders weit zur Fingerspitze hin verlängert ist. Damit wird erreicht, dass die Lage des Endgelenks 21 des Daumens fixiert wird.

Der Steg 5 in der Orthese 1C in der **Fig. 4** ist zwischen den beiden hintereinanderliegenden Ringbügeln 3,4 als verbreiterte integrierte Stützschiene 19 ausgebildet. Eine solche Orthese ist insbesondere zur Applikation an einem Daumen vorgesehen. Weiterhin sind in der Fig. 4 flächenhafte filigrane Ausnehmungen 22 sichtbar. Gemäß **Fig. 4** (und 8) können Orthesen (1C, 1E) mehrteilig ausgebildet sein. Diese weisen Gelenkmittel, z.B. das Scharnier 40 an einem Ringbügel 3,4 und/oder das Scharnier 40' im Bereich des Stegs 5 auf. Das klappbare Flügelteil 17' in Fig. 4 ermöglicht es, den Ringbügel 4 zu öffnen und zu schließen.

Mit einer mit Bezugszeichen 30 angedeuteten, den Spalt 18 überbrückenden Arretierung zwischen Flügel-Element 17' und dem anderen Flügel 17, kann das angelenkte Ortheseteil 17' im geschlossenen Zustand - mit üblichen, bekannten Rast- und Verschlussmitteln fixiert werden.

In **Fig. 5** ist eine Ansicht in Richtung der Längsachse einer Orthese dargestellt, wobei verschiedene mögliche Verformungsvorgänge durch Richtungspfeile angedeutet sind. Die vorteilhafte Unterteilung der Ringbügel 3, 4 in einen linken und einen rechten Flügel 17 ermöglicht sowohl eine Anpassung in Umfang und Durchmesser durch Abschleifen an den einander zugeordneten Stirnflächen 29 (Fig. 2 und 4) und anschließendem Zusammenpressen der Flügel 17. Darüber hinaus ist auch eine Anpassung möglich, wobei die Flügel 17 auseinander gepresst werden, wobei der Spalt der Unterbrechung 18 vergrößert wird. Sofern der Spalt für einen optimalen Sitz zu groß würde, kann auf die nächste Grundgröße des Rohlings zurückgegriffen werden, welcher dann verengt werden müsste. Somit kann mit einer geringen Anzahl von Grundgrößen der Rohlinge eine optimale Versorgung der Patienten erreicht werden.

Die waagerecht liegenden Pfeile in Fig. 5 deuten eine Verringerung oder Vergrößerung der Spaltbreite an. Die senkrecht (zur Mitte der Orthese oder von ihr weg laufenden Pfeile) geben Verformungsrichtungen an, bei denen die Spaltbreite nahezu unverändert bleibt, aber der Durchmesser sich verändert. Weiterhin sind in **Fig. 5** Schwächungslinien 34 erkennbar, die parallel zu der Richtung eines Spalts verlaufen. Die Schwächungslinien bilden eine Art Filmscharnier als Ort (Linie), in dem eine gezielte Verformung stattfinden kann. Die Schwächungslinien liegen vorzugsweise im Inneren der Orthese und sind äußerlich nicht erkennbar, und damit unauffällig.

Die **Fig. 6** zeigt schematisch eine Vielzahl möglicher Lagen von Unterbrechungen (S1 bis S7), die zusammenfassend tabellarisch abgehandelt werden. Wie schon erwähnt, können eine oder zwei solcher Unterbrechungen entweder im Ringbügel und/oder im Steg ausgebildet sein:
S1 : gerade Unterbrechung; Lage etwa parallel zur Achse (A) der Orthese, Ecken der Unterbrechung gerundet;
S2: bogenförmige Unterbrechung, seitlich am Ringbügel, schräg zur Achse der Orthese;
S3: gerade Unterbrechung, seitlich am Ringbügel, schräg zur Achse der Orthese, scharfkantige Ecken; S4: gerade Unterbrechung (Längsschlitz) im Steg, Lage etwa parallel zur Achse der Orthese;
S4': Unterbrechung S4' im Steg quer (etwa rechtwinklig) zur Achse der Orthese;
S5: gerade Unterbrechung im Steg, Lage etwa rechtwinklig zur Achse der Orthese;
S6: Unterbrechung in Zackenform; Lage etwas geneigt zur Achse der Orthese, scharfkantig am Rand auslaufend;
S7: gerade Unterbrechung, etwa auf der Linie von S1, scharfkantige Ecken am Rand des Ringbügels.

In **Fig**. **7** ist eine Ansicht gezeigt mit Blick auf die Steg-Seite der Orthese 1 E. Im Steg 5 ist eine Unterbrechung S4' quer (etwa rechtwinklig) zur Achse der Orthese vorhanden. Seitlich an der Orthese sind zwei Scharniere 40' angebracht; siehe auch Einzelheit im unteren Teil der Figur. Mit den Scharnieren 40' können die Ringbügel 3,4 relativ zueinander zum Steg 5 geöffnet und geschlossen werden. Bei besonderer Ausgestaltung des Verschlusses 30' könnte auch die Stellung (Winkellage) beider Ringbügel 3,4 relativ zueinander verstellbar sein. Mit Bezugszeichen 32' sollen zwei Magnetverschlüsse (mit je zwei einander zugewandten Kupplungsteilen) angedeutet sein. Die Fig. 7 zeigt mehrere Möglichkeiten von Verschlüssen, die jedoch nur einzeln (aber nicht gemeinsam) realisiert sein können.

### Bezugszeichenliste, auch soweit nicht in der Beschreibung

1 (A B C E) Orthese
2 Daumen und Finger
3 und 4 distaler Ringbügel (oberer) und proximaler Ringbügel (unterer)
5 Steg und Steg doppelt ausgebildet
6 und 7 distale (obere) und proximale (untere) ringförmige Öffnung
8 Fingeraußenfläche
9 Handaußenfläche
10 Fingerinnenfläche
11 Handinnenfläche
12 Hand
13 Daumengrundgelenk
13' Fingergelenk
14 Gelenkeinfassung
15 Daumensattelgelenk
16 Stützfläche
17 und 17' Flügel und klappbarer Flügel
18 Unterbrechung (Spalt)
19 integrierte Stützschiene
20 Fingerspitze
21 Fingerendgelenk
22 Ausnehmungen
24 Innenfläche der Orthese
29 Stirnfläche Flügel
30, 30' und 32' Verschluss (Ösenverschluss) und Magnetverschluss
34 Schwächungslinie
40, 40' Gelenk (Scharnier)
A Achse Orthese; Einschubrichtung des Fingers,
S1 bis S7 Beispiele von Lagen und Formen von Unterbrechungen

## Patentansprüche

1. Orthese für eine Extremität, insbesondere Finger, umfassend
- zwei einer Extremität (2,2') zugeordnete Ringbügel (3,4) und mindestens einen Steg (5), der die beiden Ringbügel (3,4) miteinander verbindet,
- wobei beide Ringbügel (3,4) für die Extremität (2,2') zwei ringförmige, hintereinander liegende Öffnungen (6,7) als Einführöffnungen bilden,
- und die Orthese (1A, 1B, 1C, 1E) durch das Zusammenwirken beider Ringbügel (4,5) mit mindestens einem Steg (5) in einer Endlage an mindestens einem Gelenk (13,15) der Extremität (2,2') festlegbar ist,
- wobei die Orthese (1A, 1B, 1C, 1E) oder zumindest einzelne Elemente der Orthese aus plastissch verformbarem Material bestehen,
- wobei die Orthese (1A, 1B, 1C, 1E) im Bereich des Stegs (5) eine durchgehende Unterbrechung (S4,S4') aufweist und/oder im Bereich mindestens eines Ringbügels (3,4) eine durchgehende Unterbrechung (18) aufweist, wodurch ein Ringbügel (3,4) in einen linken und einen rechten Flügel (17) geteilt ist und
- die Orthese (1A, 1B, 1C, 1E) durch plastische Verformung in eine unter normalen Nutzungsbedingungen unveränderbare Form an das mindestens eine Gelenk (13,13') anpassbar ist,
**dadurch gekennzeichnet, dass**
zwei parallel liegende Stege (5') ausgebildet sind, zwischen denen eine längliche, parallel zu einer Achse (A) der Orthese liegende Öffnung (25) vorhanden ist.

2. Orthese nach Anspruch 1, **dadurch gekennzeichnet, dass** die Innenform der Orthese der Außenform der Extremität angepasst ist und die Breite der Spalte (18) der Unterbrechungen so bemessen ist, dass die Ringbügel (3,4) in ihren in Finger-Ring-Größe gemessenen Durchmessern höchstens um eine ganze Finger-Ring-Größe verringerbar ist.

3. Orthese nach Anspruch 1 oder 2, **dadurch gekennzeichnet, dass** im Inneren der Orthese parallel zu der Richtung eines Spalts (18) verlaufende Schwächungslinien(34) vorhanden sind.

4. Orthese nach einem der vorhergehenden Ansprüche, **dadurch gekennzeichnet, dass** das Material einer Orthese eine Edelmetall-Legierung ist.

5. Orthese nach einem der vorhergehenden Ansprüche, **dadurch gekennzeichnet, dass** die Unterbrechung (18) in Längserstreckung (A) der Orthese gerade oder schraubenförmig verläuft.

6. Orthese nach einem der vorhergehenden Ansprüche, **dadurch gekennzeichnet, dass** die Orthese für eine Anwendung an einem Daumen ausgebildet ist.

7. Orthese nach einem der vorhergehenden Ansprüche, **dadurch gekennzeichnet, dass** der proximal liegende Ringbügel (4) in seiner parallel zur Längserstreckung (A) liegenden Breite länger ausgebildet ist als die entsprechende Breite des distal liegenden Ringbügels (3).

8. Orthese nach einem der vorhergehenden Ansprüche, **dadurch gekennzeichnet, dass** die Orthese (1A, 1B, 1C) einstückig ausgebildet ist.

9. Orthese nach einem der vorhergehenden Ansprüche, **dadurch gekennzeichnet, dass** zumindest flächenhafte Bereiche einzelner Elemente (4) der Orthese (1A, 1B, 1C, 1E) filigrane Ausnehmungen (22) oder ergonomische Ausformungen aufweisen.

## Claims

1. Orthosis for an extremity, in particular a finger, comprising
- two annular frames (3, 4) assigned to an extremity (2, 2') and at least one web (5) connecting the two annular frames (3, 4) to each other,
- wherein the two annular frames (3, 4) for the extremities (2, 2') form two annular openings (6, 7) arranged one behind the other and acting as insertion openings,
- and wherein the orthosis (1A, 1B, 1C, 1E) can be located on at least one joint (13, 15) of the extremity (2, 2') by the interaction of the two annular frames (3, 4) with at least one web (5),
- wherein the orthosis (1A, 1B, 1C, 1E) or at least individual elements of the orthosis is/are made of a plastically deformable material,
- wherein the orthosis (1A, 1B, 1C, 1E) has a continuous gap (S4, S4') in the region of the web (5) and/or a continuous gap (18) in the region of at least one annular frame (3, 4), whereby an annular frame (3, 4) is divided into a left and a right wing (17), and wherein
- the orthosis (1A, 1B, 1C, 1E) can be adapted to the at least one joint (13, 13') by plastic deformation to adopt a shape which is unchangeable in normal conditions of use,
**characterised in that**
two parallel-oriented webs (5') are formed, between which there is an oblong opening (25) extending parallel to an axis (A) of the orthosis.

2. Orthosis according to claim 1 **characterised in that** the internal shape of the orthosis is matched to the external shape of the extremity and the width of the gaps (18) is dimensioned such that the annular frames (3, 4) can, in their diameters as measured in finger ring sizes, be reduced by a maximum of one whole finger ring size.

3. Orthosis according to claim 1 or 2, **characterised in that** weakening lines (34) extending parallel to the direction of a gap (18) are provided in the interior of the orthosis.

4. Orthosis according to any of the preceding claims, **characterised in that** the material of an orthosis is a precious metal alloy.

5. Orthosis according to any of the preceding claims, **characterised in that** the gap (18) extends in a straight line or spirally in the longitudinal dimension (A) of the orthosis.

6. Orthosis according to any of the preceding claims, **characterised in that** the orthosis is designed for use on a thumb.

7. Orthosis according to any of the preceding claims, **characterised in that** the proximal annular frame (4) is greater in its width parallel to the longitudinal dimension (A) than the corresponding width of the distal annular frame (3).

8. Orthosis according to any of the preceding claims, **characterised in that** the orthosis (1A, 1B, 1C) is designed as a single piece.

9. Orthosis according to any of the preceding claims, **characterised in that** at least planar regions of individual elements (4) of the orthosis (1A, 1B, 1C, 1E) have filigree openings (22) or ergonomic shapings.

## Revendications

1. Orthèse pour une extrémité, en particulier un doigt, comprenant
- deux étriers annulaires (3, 4) affectés à une extrémité (2, 2') et au moins une partie médiane (5) qui relie les deux étriers annulaires (3, 4) l'un à l'autre,
- les deux étriers annulaires (3, 4) pour l'extrémité (2, 2') formant deux orifices (6, 7) annulaires situés l'un derrière l'autre en forme d'orifices d'introduction,
- et l'orthèse (1A, 1B, 1C, 1E) pouvant être fixée par la coopération des deux étriers annulaires (4, 5) au moyen d'au moins une partie médiane (5) en position finale sur au moins une articulation (13, 15) de l'extrémité (2, 2'),
- l'orthèse (1A, 1B, 1C, 1E) ou au moins des éléments individuels de l'orthèse sont constitués d'un matériau pouvant subir une déformation plastique,
- l'orthèse (1A, 1B, 1C, 1E) dans la zone de la partie médiane (5) présente un interstice continu (S4, S4') et/ou présente dans la zone d'au moins un étrier annulaire (3,4) un interstice continu (18), qui sépare un étrier annulaire (3,4) en une aile gauche et droite (17) et
- l'orthèse (1A, 1B, 1C, 1E) pouvant être adaptée par déformation plastique en une forme invariable dans des conditions d'utilisation normales à au moins une articulation (13, 13'),
**caractérisée en ce que** sont conçues deux parties médianes (5') parallèles, entre lesquelles se trouve un orifice (25) allongé disposé parallèlement à un axe (A) de l'orthèse.

2. Orthèse selon la revendication 1, **caractérisée en ce que** la forme intérieure de l'orthèse est adaptée à la forme extérieure de l'extrémité et la largeur des écartements (18) des interstices est calculée de telle sorte que les étriers annulaires (3, 4) dans leur diamètre mesuré en taille de bague peut être réduit tout au plus d'une taille de bague entière.

3. Orthèse selon la revendication 1 ou 2, **caractérisé en ce qu'**à l'intérieur de l'orthèse se trouvent deux lignes d'affaiblissement (34) s'étendant parallèlement à la direction d'un écartement (18).

4. Orthèse selon l'une quelconque des revendications précédentes, **caractérisée en ce que** le matériau d'une orthèse est un alliage de métal noble.

5. Orthèse selon l'une quelconque des revendications précédentes, **caractérisée en ce que** l'interstice (18) s'étend de manière rectiligne ou hélicoïdale dans le sens de la longueur (A) de l'orthèse.

6. Orthèse selon l'une quelconque des revendications précédentes, **caractérisée en ce que** l'orthèse est conçue pour être utilisée sur un pouce.

7. Orthèse selon l'une quelconque des revendications précédentes, **caractérisée en ce que** l'étrier annulaire (4) proximal est plus long dans sa largeur parallèle à l'étendue longitudinale (A) que la largeur correspondante de l'étrier annulaire (3) distal.

8. Orthèse selon l'une quelconque des revendications précédentes, **caractérisée en ce que** l'orthèse (1A, 1B, 1C) est conçue en un seul tenant.

9. Orthèse selon l'une quelconque des revendications précédentes, **caractérisée en ce qu'**au moins des zones planes des éléments individuels (4) de l'orthèse (1A, 1B, 1C, 1E) présentent des évidements filigranés (22) ou des déformations ergonomiques.
